# EUROPEAN PATENT SPECIFICATION

(11) **EP 1 105 185 B1**
(45) Date of publication and mention of the grant of the patent: **09.06.2004**
(21) Application number: 99942244.7
(22) Date of filing: 17.08.1999
(51) Int. Cl.: A61N 1/04

(54) **ELECTRODES**
ELEKTRODEN
ELECTRODES

(30) Priority: 17.08.1998 JP 23059498
(43) Date of publication of application: 13.06.2001
(73) Proprietor: MINNESOTA MINING AND MANUFACTURING COMPANY, St. Paul, Minnesota 55133-3427 (US)
(72) Inventor: TAKAKI, Shunsuke, Sagamihara-city, Kanagawa pref. 229 (JP); SUWA, Toshihiro, Sagamihara-city, Kanagawa pref. 229-1131 (JP)
(74) Representative: Hermann, Gerhard, Dr.
(86) International application number: PCT/US1999/018654
(87) International publication number: WO 2000/009203

(56) References cited:
- EP-A- 0 138 347
- WO-A-95/20634
- FR-A- 2 509 182
- US-A- 5 438 988

## Description

### Field of the Invention

The invention relates to electrodes, and particularly biomedical electrodes, having a conductive adhesive layer for attaching the electrode to an adherend (like mammalian skin), wherein a portion of the conductive adhesive layer is treated to provide improved adhesion to the adherend over the untreated portion of the conductive adhesive layer.

### Background of the Invention

Heretofore, there has widely been used a biomedical electrode comprising an electrode supporting substrate, a stud-shaped, Ag/AgCl conductor, and a cohesive and non-adhesive conductive gel composition such as that disclosed in Unexamined Patent Publication (Kokai) No. 56-501108, WO81/00785, U.S. Patent No. 4,406,827. Since the conductive gel has no adhesion strength, a wide backing tape was required for fixing the electrode to the adherend.

Certain hydrophilic conductive adhesives as represented by Patent No. 2625179, Examined Patent Publication (Kokoku) No. 8-193494 and Patent Publication (Kokai) No. 7-501101 have been developed. These materials made it easier to apply and fix electrodes without using a backing tape for fixation. These conductive adhesives have adequate initial adhesion strength, but absorption of sweat from skin can reduce the adhesive's adhesion strength, and the edge portion of the electrode may delaminate from the skin. Adsorption of large quantities of sweat into the adhesive can convert the conductive adhesive into a gel state and the resulting adhesive may have essentially no adhesion strength. In such instances, the electrode easily moves on the skin which creates artifact noise, thereby obscuring readouts. Intense body movement of the subject, such as is sometimes the case when an electrocardiogram is taken, can create so much noise that a clear electrocardiogram can not be recorded.

Patent Publication (Kokai) No. 9-509196 describes a very useful conductive adhesive that has a hydrophilic phase and a hydrophobic phase and good physical properties. The hydrophilic phase of the adhesive contributes to the conductivity of the adhesive but it does not contribute as greatly to the adhesion strength of the adhesive as does the hydrophobic phase. Accordingly, conductivity and adhesiveness must be balanced when formulating the adhesive for a particular electrode application. When conductivity of the adhesive must be very high, the hydrophilic phase may need to constitute a larger fraction of the adhesive, and this can adversely impact the long term adhesion strength of the adhesive. Accordingly, it would be useful to improve the adhesion strength of hydrophilic/hydrophobic phase adhesives to improve the long term wear properties of electrodes made using such adhesives without compromising electrode conductivity.

Document FR-A-8 212 085 relates to a iontophoresis device with a conducting layer of gel, which can adhere to the human skin.

### Brief Description of the Drawings

Fig. 1 is a cross-sectional view of one electrode embodiment of the invention that comprises a conductor in the form of a sheet or layer.
Fig. 2 is a cross-sectional view of another electrode embodiment of the invention that comprises a conductor in the form of a stud.
Fig. 3 is a perspective view showing one embodiment of a heat block that can be used to hot-press the conductive adhesive in the present invention.
Fig. 4 is a plan view of yet another electrode embodiment wherein the perimetrical portion of a conductive adhesive layer was hot-pressed using the heat block of Fig. 3 to provide a groove or depression between the hot-pressed portion of the conductive adhesive layer and the non-hot-pressed portion.

### Summary of the Invention

In one aspect, the invention provides an electrode adapted for wear on an adherend, such as mammalian skin, as disclosed in cl. 1.

In preferred embodiments of the electrode, the electrode support supports a conductor either: (i) in the form of a layer of conductive material (e.g., conductive ink) disposed or coated upon at least a portion of the first opposed surface of the electrode support, or, (ii) in the form of a conductive stud that passes completely through a portion of the electrode support to project below the second opposing surface of the electrode support. The conductive adhesive layer is disposed upon a major portion of the first, opposed surface of the electrode support such that it covers at least a major portion (and preferably essentially all) of the surface of the conductor whether in the form of a layer, or a stud. In some embodiments, the stud may also project above the first opposing surface of the electrode support.

Preferably, a release liner is provided upon the surface of the conductive adhesive layer to protect the exposed surface of the adhesive layer until the electrode is ready to be applied to an adherend. Prior to applying the electrode to an adherend, the release liner is removed to expose the conductive adhesive layer.

In another aspect, the invention provides a method of improving the adhesion properties of a coating or layer of a conductive adhesive composition as disclosed in cl. 15.

### Detailed Description of the Invention

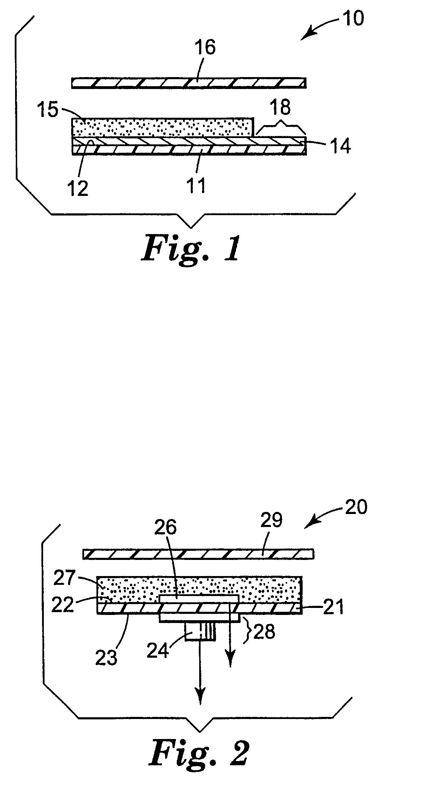

Figure 1 shows one electrode embodiment not part of the invention. The electrode 10 comprises an electrode support 11 having two, opposed surfaces 12, 13, wherein a conductor 14, in the form of a sheet or layer of conductive material (e.g., a conductive ink coating), is disposed upon a portion of a first, opposed electrode support surface 12. Preferably, the area of the conductor 14 is coextensive with the area of the first, opposed electrode support surface 12. Provided on a portion of the exposed surface of the conductor 14, is a layer of a conductive adhesive 15. A release liner 16 is laminated to the exposed surface of the conductive adhesive layer 15. A portion of the conductor 14 that is not coated with conductive adhesive, together with an adjacent portion of the electrode support 11, forms a tab 18 for attachment to a lead wire (not shown). A portion, and preferably a perimetrical portion, of the conductive adhesive layer 15 is hot-pressed according to the method described herein to improve its adhesion properties over the properties of the untreated portion of the conductive adhesive layer 15.

Figure 2 shows another electrode embodiment not part of the invention wherein the electrode 20 comprises an electrode support 21 with two, opposed surfaces 22, 23, and a conductor 24 in the form of a conductive stud. The conductor 24 passes through a portion of a first, opposed electrode support surface 22, and completely through the electrode support 21 to project below the second, opposed electrode support surface 23. A layer of conductive adhesive is provided both over the first, opposed surface of the electrode support 22, and the surface of a conductor portion 26 that projects above the electrode support surface 22 to form a conductive adhesive layer 27. The surface of a second conductor portion 28 projecting below the second, opposed electrode support surface 23, serves as a connector for a lead wire (not shown). A portion, and preferably a perimetrical portion, of the conductive adhesive layer 27 is hot-pressed according to the method described herein to improve the adhesion properties of the hot-pressed portion. Also shown is a release liner 29 disposed upon the exposed surface of the conductive adhesive layer 27.

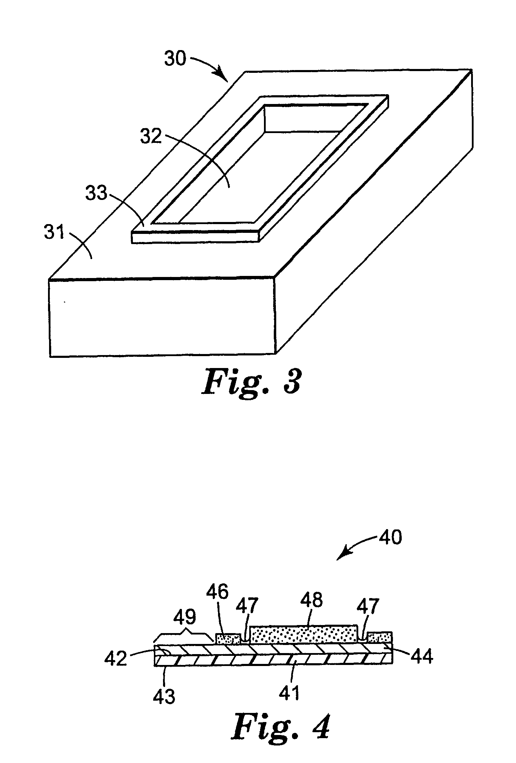

In the heat-pressing process, heat can be applied directly to the conductive adhesive layer, or indirectly to the release liner. Typically, a heated block with a small area, such as that on a micro-press, is used to apply heat. An embodiment of a machined, metal block useful in heat pressing the conductive adhesive coating is shown in Figure 3. The block 30 has a rectangular, outer portion 31 and a cut-out 32. Disposed around the entire perimeter of the cut-out 32, is a raised projection 33 which can be of various shapes other than the one depicted in Figure 3. The block 30 is heated to the desired temperature for example, by using a continuous or impulse heat source, ultrasonic waves, a radio frequency heat source, a lamp heater or the like. The temperature the block is heated will depend upon the formulation of the adhesive, and the thickness of the adhesive coating, but preferably, the block should be heated at a temperature from about 50 to 200°C, and preferably from 90 to 170°C. A temperature, which causes mobility of the surfactant in conductive adhesive, is preferred. The heated block is then applied to the conductive adhesive layer with a pressure of about 0.5 to 4 kg/cm². The pressure should be selected so as not to break the conductive adhesive layer or to squeeze the adhesive layer off of the electrode. The pressing time should be selected based on the type of materials used in the construction of the electrode and the temperature of the heater. Typical pressing times are from about 0.1 to 10 seconds, and preferred times are about 1 to 5 seconds. When the pressing time is too long, the adhesion strength of the adhesive can be adversely affected.

Preferably, a heating block similar to that shown in Figure 3 is used to heat press the conductive adhesive layer. The block is positioned over the conductive adhesive layer such that the portion of the layer to be heat pressed is located under the outer portion 31, and the portion of the layer that is not to be heat pressed is located under the cut-out 32. An electrode having a structure similar to that depicted in Fig. 4 (shown without release layer) will result. The electrode 40 comprises an electrode support 41 with two, opposed surfaces 42, 43. Disposed on a first, opposed surface of the electrode support 42, is a sheet-like conductor 44 that is preferably coextensive with the area of the first, opposed electrode support surface 42. Disposed upon the conductor 44, is a conductive adhesive layer 45 that was heat pressed using the heat block shown in Fig. 3. The conductive adhesive layer has a heat-pressed perimetrical portion 46, which underlied the outer portion 31 of the heat block 30 during heat pressing. The conductive adhesive layer also has a groove or indentation 47 that is adjacent to the inner perimeter of the heat-pressed perimetrical portion 46, and the outer perimeter of a non-heat pressed portion of the conductive adhesive layer 48. The non-heat-pressed portion underlied the cut-out 32 of the heat block 30 during heat pressing. The groove 47 was formed by the projection 33 on the heat block 30. Also provided is a tab portion 49 that is not coated with conductive adhesive and is formed from the adjacent portions of the electrode support 41 and the conductor 44. The groove helps prevent the migration or diffusion of water from the non-heat pressed adhesive portion 48 into the heat pressed adhesive portion 46, thereby helping to maintain the improved adhesive properties obtained by heat pressing. Preferably, the volume of the groove or indentation should amount to less than about 10% of the volume of the non-heat pressed portion of the conductive adhesive layer. Preferably, such an indentation can be obtained by using a block with a convex protrusion 0.1 to 2 mm in height, and for an adhesive coating about 1 mm thick, a heat block protrusion of about 0.4 to 0.6 mm in height will form a clear groove between the hot pressed and non-heat pressed portions of the conductive adhesive layer. Alternatively, the formation of the groove and the heat pressing of the adhesive can be conducted in separate steps, although combining the steps as previously described is preferred.

If too great an area of the adhesive conductive layer is heat-pressed, the adhesion of the electrode to the skin can be too great and can cause pain when the electrode is removed from the skin. On the other hand, the heat-pressed adhesive area must be large enough to provide the desired adhesive wear properties for the electrode. Preferably, adhesion strength should be greater than 110 g (as measured in a 180 degree peel test using a sample width of 25.4 mm and a peel rate of 300 mm/min.) and less than an amount that will cause pain when the electrode is removed from the skin. Generally, 180 degree peel test values greater than about 500 g (measured according to the aforementioned test parameters) will result in painful electrode removal. Accordingly, 180 degree peel test values of about 110 to 500 g are preferred, and most preferably, values are about 150 to 500 g. Because heat pressing can also reduce the water content of the conductive adhesive and impair the adhesive's conductivity, the heat-pressed area must be selected such that an acceptable overall conductivity for the conductive adhesive layer is maintained. Preferably, the absolute area of the heat-pressed portion of the conductive adhesive layer is at least about 1 cm². More preferably, the heat-pressed portion will be between about 1 to 4 cm². It is preferred to heat press a perimetrical portion of the conductive adhesive layer because this increases the adhesive properties of the part of the electrode typically subjected to the most delaminating force (e.g., such as lead wire tension). Preferably, the width of the heat-pressed perimetrical portion for an electrode designed for short wearing periods will be about 1 to 3 mm. The heat-pressed, perimetrical portion width for electrodes designed for long wear, or for situations in which the subject will move during wear (e.g., stress tests), is preferably about 5 to 20 mm.

Heat pressing may also be carried out by applying heat to the conductive adhesive layer in a heated pattern (e.g., lattice patterns, wave patterns, etc.)...

Preferably, pinholes are introduced into the release liner prior to or during heat-pressing to permit the water vapor evolving during heat pressing to escape. The pinholes can be introduced prior to heat-pressing by piercing the release liner, and, if desired, the underlying layers of the electrode, using a tool having a multiplicity of needle-like protrusions. The pierced area should approximately correspond to the area of the conductive adhesive layer that is to be heat-pressed. Alternatively, pinholes can be introduced during the heat-pressing process by using a heat block with a multiplicity of needle protrusions located in the region of the block that will overly the portion of the conductive adhesive coating to be heat-pressed.

The electrode support can be selected from any flexible plastic film useful as a support for biomedical electrodes. Various commercially available plastic sheets about 10 µm to 1 mm thick may be used.

The conductor can be made of any of the conductive materials useful as conductors in biomedical electrodes. Various stud shaped and sheet-like conductors are known in the art and are suitable for use in the invention. Single or multilayer, sheet-like conductors may be used in the invention. One example of a useful conductor is a sheet coated with ink containing Ag and AgCl. An example of a commercially available sheet-like conductor that is suitable for use in the invention is MSX-4510 manufactured by Minnesota Mining and Manufacturing Company, St. Paul, Minnesota, U.S.A. Other useful sheet-like conductors are described in PCT/US96/05938 and U.S. Patent No. 5,924,983 (which descriptions are herein incorporated by reference), where a two-layer conductive sheet comprising: (a) a low porosity base conductor layer made of a conductive coating composition containing a hydrophobic polymer binder and carbon particles and, optionally, Ag particles, and (b) a porous top conductor layer, formed on the base conductor layer, that is made of a conductive coating composition containing a polymer binder, Ag/AgCl particles and carbon particles.

Any release liner, such as those obtained by coating a paper sheet or plastic film, that are useful in biomedical electrodes, can be used in this invention. Typically, such materials are plastic or paper sheets coated or impregnated with a material that imparts or enhances the release properties of the sheet.

The conductive adhesive layer comprises, and preferably consists essentially of, or consists of, a hydrophilic phase and a hydrophobic phase. Particularly preferred examples of such adhesives are disclosed in the Japanese Patent Publication (Kokai) No. 9-509196, WO95/20634, and U.S. Patent Nos. 5,409,966 and 5,438,988 (which descriptions are incorporated by reference herein). A hydrophilic polymer material, an electrolyte, and a humectant for preventing evaporation of the electrolyte and water are generally contained in the hydrophilic phase. As the hydrophilic polymer material, a polyethylene glycol group-containing material having good ion conductivity, for example, methoxypolyethylene glycol or polymer derived from N-vinylpyrrolidone may be used. Such a hydrophilic polymer can be obtained by adding an ultraviolet polymerization initiator (e.g., 1-(4-(2-hydroxyethoxy)-phenyl)-2-hydroxy-2-methoxy-1-propane-1-one, 4-trimethylaminomethylbenzophenone hydrochloride, etc.) to a monomer or oligomer for the hydrophilic polymer material and performing the polymerization reaction. As the electrolyte, an aqueous solution of potassium chloride, sodium chloride, lithium chloride or the like may be used. As the humectant, propylene glycol, sodium DL-pyrrolidonecarboxylate or the like can be used. A hydrophobic polymer material is contained in the hydrophobic phase. As the hydrophobic polymer, for example, there may be used copolymers of acrylic acid and isooctyl acrylate, 2-ethylhexyl acrylate, n-butyl acrylate and the like. Such a hydrophobic polymer can be obtained by polymerizing the monomer or oligomer for the hydrophobic polymer, in a surfactant stabilized hydrophobic phase, that is contained in a hydrophilic phase. The polymerization can be carried out by exposing the polymerizable composition to UV radiation. In such a case, an ultraviolet polymerization initiator for hydrophobic phase should be included in the polymerizable composition. As the above surfactant, there can be used polyoxyethylene oleyl ether, ammonium salt of α-sulfo-ω-(1-((nonylphenyl)methyl-2-(2-propenyloxy)ethoxy-(polyoxy-1,2-ethanediyl)), ammonium salt of sulfated polyoxyethylene alkyl ether and sodium salt of sulfosuccnic alkyl(C₈₋₂₀) alkenyl(C₃₋₄) ester. Examples of ultraviolet polymerization initiators for the hydrophobic phase include: 1-hydroxy-cyclohexyl-phenylketone and 2,2-dimethoxy-2-phenylacetophenone.

The conductive adhesive can be produced in the following manner. The aforementioned components of the hydrophilic phase and hydrophobic phase are mixed to prepare a liquid composition. This composition is purged with a nitrogen gas to drive out dissolved oxygen. A reinforcing material, such as a non-woven fabric, is placed on a silicone-coated surface of a transparent polyester liner, and, after impregnating it with or applying the liquid to the reinforcing material at a thickness of about 0.2 to 2 mm, another layer of polyester liner is placed on the reinforcing material such that its silicone-coated surface is brought into contact with the liquid. The composite is then exposed to UV radiation (e.g., 500 to 3000 mJ) to effect the desired level of cure in the adhesive. Subsequently, one of the polyester liner layers is peeled off and the conductive adhesive layer is laminated to the conductor and electrode support composite. The polyester liner that remains adhered to the other side of the conductive adhesive layer can be left adhered to the adhesive layer such that it becomes the release liner of the electrode. Thereafter, the electrode can be hot pressed. Before or after the hot pressing step, the edge of the electrode can be trimmed (where the conductor is in sheet form) to form a tab which serves as an attachment for a lead wire.

The following examples are offered to aid in the understanding of the present invention and are not to be construed as limiting the scope thereof. Unless otherwise indicated, all parts and percentages are by weight.

### Examples 1 to 3 and Comparative Example C1

### Preparation of Electrodes

A biomedical electrode was produced by forming a conductor layer and a conductive adhesive layer on a flexible plastic film support layer. The conductor layer was prepared according to the process described in U.S. Patent No. 5,924,983, which description is incorporated by reference herein. First, a 15 µm thick, sheet-like conductor having porosity (an adsorption surface area of nitrogen gas per unit area as measured by the BET method of 4.8 m²/m²) was formed on a transparent, 75 µm thick, polyester film (available from the Unitika Co. as "Emblet") by applying mixed ink for a base conductor layer and drying the coated film. The mixed ink for the base conductor layer was prepared by combining 98.5 parts of a first ink, 1 part of an Ag/AgCl ink available from Ercon Co. as "R-301"; and 0.5 parts of a curing agent available from Dow Chemical Co. as "PAPI 2027", using a mixer. The first ink which was prepared by combining 5.7 wt. percent of a conductive carbon having small porosity (BET value: 50 m² /g) available from Mitsubishi Chemical Co. as "#3050B"; 2.5 wt. percent of a conductive carbon having large porosity (BET value: 980 m²/g) available from Akzo Co. as "Ketchin Black EC".; 13.3 wt. percent of a urethane binder available from BF Goodrich Co. as "Estane 5703"; 47.5 wt. percent of a propylene glycol monomethyl ether acetate; 24.8 wt. percent of methyl ethyl ketone; and 6.2 wt. percent of toluene as a solvent. Then, an 8 µm thick, porous, top conductor layer (adsorption surface area of nitrogen gas per unit area measured by BET method: 24 m²/m²) was formed on the base layer only on the portion that would be in contact with the conductive adhesive by applying mixed ink for the top conductor layer and drying the coating. The ink for the top conductor layer was prepared by combining: 84.5 parts of a second of ink, 15 parts of an Ag/AgCl ink available from Ercon Co. as R-301; and 0.5 parts of a curing agent available from the Dow Chemical Co. as "PAPI 2027", using a mixer. The second ink was prepared by combining: 5.2 wt. percent of conductive carbon having large porosity (BET value: 980 m²/g available from Akzo Co. as "Ketchin Black EC"), 9.6 wt. percent of a urethane binder available from BF Goodrich Co. as "Estane 5703", 30.6 wt. percent propylene glycol monomethyl ether acetate, 43.7 wt. percent of methyl ethyl ketone, and 10.9 wt. percent of toluene as a solvent.

Drying of the base conductor layer was performed at the temperature between about 100 to 110°C and an air flow of 9 m/sec, and drying of the top conductor layer was performed at temperatures between about 150 to 160°C and an air flow of 1.5 m/sec. Consequently, a conductor sheet comprising a polyester film, a base conductor layer with small porosity and relatively small Ag/AgCl content, and a top conductor layer with large porosity and relatively large Ag/AgCl content, was obtained.

On the conductor sheet, a conductive adhesive coating consisting of a hydrophilic phase and a hydrophobic phase was formed by mixing raw materials in the proportion of the following table and performing the polymerization reaction.

**Table 1**

| | | Raw Materials | Example 1 (wt. %) | Example 2 (wt.%) | Example 3 (wt. %) |
|---|---|---|---|---|---|
| Hydrophobic Phase | Ultraviolet polymerization initiator | Irg. 184 | 0.05 | 0.07 | 0.06 |
| | Monomer for hydrophobic polymer material | AA | 13.92 | 15.43 | 13.75 |
| | | IOA | 13.92 | 22.13 | 19.72 |
| | Surfactant | Brij 97 | 17.9 | -- | -- |
| | | SE-10N | -- | 16.1 | |
| | | JS-2* | -- | -- | 46.6 |
| Hydrophilic Phase | Monomer for hydrophilic polymer material | MPEG-550MA | 13.92 | 2.68 | 5.5 |
| | | AM90G | -- | 2.68 | -- |
| | Humectant | PPG | 9.95 | -- | -- |
| | | PCA-Na | -- | 6.71 | 6 |
| | Electrolyte | 4% KCI | 29.84 | 33.53 | 7.77 |
| | Ultraviolet polymerization initiator | Irg. 2959 | 0.5 | 0.67 | 0.6 |
| | | Total | 100 | 100 | 100 |

| | | | | | |
|---|---|---|---|---|---|
| * Water content of JS-2 is 62 wt. %. | | | | | |

Acrylic acid (AA) and isooctyl acrylate (IOA) monomers and surfactant were combined, followed by polymerization using an ultraviolet polymerization initiator. As the surfactant, a polyoxyethylene oleyl ether ("Brij-97") available from Sigma Co. U.S.A. as "Brij-97", was used in Example 1; an ammonium salt of α-sulfo-ω-(1-((nonylphenyl)methyl-2-(2-propenyloxy) ethoxy-(polyoxy-1,2-ethanediyl)) ("SE-10N"), available from Asahi Denka Chem Co. as "Adekareasorp SE-10N", was used in Example 2; and a sodium salt of sulfosuccnic alkyl (C₈₋₃₀) alkenyl (C₃₋₄) ester ("JS-2"), available from Sanyo Kasei Kogyo Co. as "Eleminol", was used in Example 3. 1-hydroxy-cyclohexyl-phenylketone ("Irg. 184"), available from Ciba Geigy Co. as "Irgacure 184", was used as the ultraviolet polymerization initiator for hydrophobic phase. The monomer methoxy polyethylene glycol 550 monoacrylate ("MPEG-550MA"), available from Satormer Chem. USA, was used to prepare the hydrophilic polymer material. In Example 2, an additional monomer, methoxy polyethylene glycol monoacrylate "AM-90G", available from Shinnakamura Chem. Co. as "AM-90G", was also used to prepare the hydrophilic polymer material.

As the humectant, either propylene glycol ("PPG"), or sodium DL-pyrrolidonecarboxylate solution ("PCA-Na"), available from Ajinomoto Co. as "PCA-Na", were used. As the ultraviolet polymerization initiator for hydrophilic phase, 1-(4-(2-hydroxyethoxy)-phenyl)-2-hydroxy-2-methoxy-1-propane-1-one ("Irg. 2959"), available from Ciba Geigy Co. as "Irgacure 2959", was used.

A liquid mixture containing the above-described materials for the hydrophobic and hydrophilic phases was prepared. The resulting liquid was purged with a nitrogen gas to drive out dissolved oxygen, and a nonwoven fabric (available from Kimberly Clark Co. as "KC Tissue") was placed on a silicone-coated surface of a transparent polyester liner. After impregnating the fabric with the liquid to a thickness of 1 mm, another layer of the polyester liner was placed on top of the liquid coating, such that its silicone surface is brought into contact with the liquid. The resulting composite was cured by exposing it to UV radiation (about 2400 mJ/cm²) using a low-pressure mercury lamp. Consequently, a 1 mm thick conductive adhesive layer consisting of a hydrophilic phase and a hydrophobic phase was obtained.

### Evaluation of Adhesive Conductivity

Subsequently, the polyester liner sheets were peeled from the conductive adhesive layer, and the conductive adhesive layer was laminated to the conductor such that the top conductor layer contacted the conductive adhesive. Thereafter, the composite was cut to provide a conductive adhesive area of 5.5 cm² and a tab portion area of 1.6 cm². Identical electrodes were laminated together and tested using the electrode characteristic test of the AAMI Society, U.S.A. The results of the conductivity tests are provided in Table 2 along with conductivity test results (Comparative Example C1) for a conductor layer/adhesive layer composite of the same construction except that a conductor adhesive commercially available from Minnesota Mining and Manufacturing Company, St. Paul, MN as RD-40 was used instead of a conductive adhesive with a hydrophobic phase and a hydrophilic phase. The data shows that the electrodes according to the invention have sufficient conductivity to be useful as biomedical electrodes.

**Table 2**

| Test Measurements | AAMI specification | Example 1 | Example 2 | Example 3 | Comparative Example C1 |
|---|---|---|---|---|---|
| DC offset voltage | ≤ 100 mV | 0.2 mV | -0.1 mV | -0-1 mV | -0.5 mV |
| AC impedance | ≤ 2000 Ω | 500 Ω | 499 Ω | 400 Ω | 422 Ω |
| Offset potential after 5 seconds since the completion of defibrillation | ≤ 100 mV | 2.2 mV | 9.3 mV | 14.3 mV | 24.1 mV |
| Recovery rate after 5 seconds since the completion of defibrillation | 0 to -1.0 mV/s | -0.3 mV/s | -0.3 mV/s | -0.5 mV/s | -0.6 mV/s |

### Evaluation of Adhesive Peel Strength

Electrodes of the same laminate constructions were prepared having conductive adhesive coating areas of 6.3 cm² and tab portions of 2.5 cm² . The width of the resulting electrodes was 2.54 cm. These electrodes were applied to the lower arm of a human body and pressed by a 2 kg roller. Then 180 degree peel tests were run at a rate of 300 mm/min to determine bond strength. The results are shown in Table 3. The data shows that the electrodes had acceptable peel strengths.

**Table 3:**

| 180 degree peel test | |
|---|---|
| Example No. | Peel strength (g/inch) |
| 1 | 115 |
| 2 | 182 |
| 3 | 210 |
| C1 | 247 |

The biomedical electrodes produced as described above were hot-pressed in the following manner. In each of the electrodes, pinholes having a pore diameter of 0.5 mm were formed through the entire surface of the release liner. The holes were spaced about 3 mm apart. The whole surface of the adhesive was hot-pressed by applying a hot press to the release liner at various temperatures under a pressure of 1 kg/cm² for 5 seconds. Each electrode was applied to the lower arm of a human body and pressed on to the skin. The 180 degree peel strength values were immediately measured by peeling the electrode from the skin at a rate of 300 mm/min. The results are provided in Table 4.

**Table 4**

| | Peel Strength | | | |
|---|---|---|---|---|
| Temperature (°C) | Example 1 (g/in) | Example 2 (g/in) | Example 3 (g/in.) | C1 (g/in.) |
| Untreated | 115 | 182 | 210 | 247 |
| 43 | 148 | 190 | 237 | 238 |
| 65 | 193 | 280 | 275 | 233 |
| 93 | 298 | 410 | 485 | 230 |
| 98 | 358 | 380 | 545 | 222 |
| 129 | 520 | 380 | 428 | 210 |
| 165 | 480 | 250 | 373 | 188 |
| 171 | 137 | 220 | 288 | 160 |

The data in Table 4 shows that the peel strength of the conductive adhesive increases as the hot press temperature increases up to a maximum hot press temperature. The optimal hot pressing temperature appears to depend upon the surfactant used, but many effective temperatures are found out within the range from 90 to 170°C. Furthermore, hot press temperatures greater than 170°C are generally not preferred because high temperatures can deteriorate the other components of the electrode (e.g., release liner or electrode support). An increase in adhesion strength was not achieved by hot pressing the adhesive R-40D (see C1).

Electrodes identical to Example 1, Example 2 and Comparative Example C1 electrodes were prepared as described above, except that the electrode width was 22 mm, the area of the conductive adhesive was 5.5 cm², the area of the tab was 1.6 cm², and the thickness of the conductive adhesive was about 1 mm. Thereafter, some of the electrodes were hot-pressed on the release liner side at 125°C and under a pressure of 2 kg/cm² for 1.4 seconds using a heat block with a protrusion 0.6 mm high for forming a groove. The protrusion formed grooves in the adhesive layer 1 mm wide, that separated perimetrical, hot-pressed adhesive portions from central, untreated, adhesive portions. The perimetrical, hot-pressed portions were about 1.5 mm wide. After pressing, the thickness of the perimetrical, hot-pressed adhesive portions were about 0.5 mm. Two, identical, non-hot-pressed electrodes and two, identical, hot-pressed electrodes, respectively, were laminated together and the electrode characteristic test of AAMI Society, U.S.A. was performed on each pair of laminated electrodes. Although the effective area of conductive adhesive layer of the hot-pressed electrode was reduced to about 61.8% of that of the untreated electrodes, no deterioration of electrical characteristics were measured. One of the non-hot-pressed electrodes, one of the hot-pressed electrodes and an electrode of identical construction except that RD-301 conductive adhesive was used instead of the hydrophilic/hydrophobic phase adhesive were applied to the lower arm of a human body and pressed by a 2 kg. Roller. The 180 degree peel strength for each electrode was immediately measured using a peel rate of 300 mm/min. The results are provided in Table 5. The data shows that the peel strength of electrodes prepared with the hydrophilic phase/hydrophobic phase adhesive improved when the adhesive was heat-pressed.

**Table 5**

| Ex. No. | No Hot Press Peel Strength (g/in) | Hot Press Peel Strength (g/in) |
|---|---|---|
| 1 | 138 | 162 |
| 2 | 230 | 313 |
| C1 | 317 | 247 |

### Example 4

An electrode was prepared by laminating a 1 mm thick conductive adhesive coating (identical to Example 1) to a conductor on a plastic film, and laminating a matted polyethylene tape (commercially available from Minnesota Mining and Manufacturing Co. U.S.A. as "Blenderm") to the plastic film surface of the tab portion on the opposite side of the conductor layer, and cutting the resulting laminate so that the electrode width was 34 mm, the area of the conductive adhesive was 13.6 cm² and the area of the tab portion was 1.44 cm². Then, the tab portion was removed, and 0.5 mm diameter pinholes were formed in the laminate by piercing through the release layer in the 3 mm wide portion of the laminate to be hot pressed.

Thereafter, a heat block with a 0.6 mm high protrusion height for forming a groove was used to hot press the laminate and form a groove. In the transverse direction of the electrode, the width of the hot-pressed portion of the adhesive coating was 5 mm. In the longitudinal direction, the width of the hot pressed portion was 8 mm. The thickness of the peripheral, hot pressed portion of the adhesive layer was 0.4 mm. The width of the groove in the adhesive layer was 1 mm. The hot pressing was performed by pressing either the electrode support or the release liner at a pressure of 2 kg/cm² for 1.4 seconds under several temperature conditions. Two, non-hot-pressed electrodes and two, hot-pressed electrodes, respectively, were laminated together and an electrode characteristic test of AAMI Society, U.S.A. was performed on each laminated pair. Although the effective area of the hot-pressed conductive adhesive was reduced to about 32.4% of that of the untreated electrode, no deterioration of electrical characteristics was measured. The non-hot-pressed electrode and hot-pressed electrode were each applied to the lower arm of a human body and pressed by a 2 kg. roller. The 180 degree peel strength values for each electrode were immediately measured using a peel rate of 300 mm/min. The results are provided in Table 6.

**Table 6**

| | 180° Peel Strength (g/34 mm width) | | | | | |
|---|---|---|---|---|---|---|
| Surface to be hot-pressed | No Heat Press | 81°C Heat Press Temp | 96°C Heat Press Temp | 110°C Heat Press Temp | 125°C Heat Press Temp | 140°C Heat Press Temp |
| Electrode Support Layer | 118 | 130 | 140 | 155 | 190 | 140 |
| Release Liner | 118 | 150 | 165 | 185 | 215 | 150 |

Regardless of the surface that is hot-pressed, the adhesion strength improved by hot pressing up to about 125°C, and good adhesion improvement can to be obtained by applying the hot press to the release liner surface.

To examine the effect of pinholes, a pinhole-treated electrode and non-pinhole-treated electrode were hot-pressed on the release liner surface at temperatures of 110°C and 125°C. The skin adhesion strength of the electrode was measured in the same manner. The results are shown in Table 7. Good results were obtained when the pinhole treatment was performed.

**Table 7**

| | 180° Peel Strength (g/34 mm) | | |
|---|---|---|---|
| | No Heat Press | 110°C Heat Press | 125°C Heat Press |
| No pinhole, treatment | 118 | 110 | 185 |
| Pinhole treatment | 118 | 160 | 215 |

To examine the effect of the hot press pressure and the effect of the height of the protrusion during formation of the groove, the adhesion strength of the electrode obtained by hot-pressing the pinhole-treated electrode from the release liner surface using a heat block at the temperature of 125°C using various pressures and protrusion height was measured in the same manner. The degree of "squeeze-out" of the adhesive from the edge of the electrode after the heat press treatment is shown in Tables 8, 9 and 10.

**Table 8**

| | Heat Press Pressure (kg) | | | | | |
|---|---|---|---|---|---|---|
| | No Heat Press | 0.5 | 1.0 | 1.5 | 2.0 | 3.0 |
| Peel strength (g/width of electrode: 34 mm) | 118 | 150 | 220 | 190 | 185 | -- |
| Squeeze-out amount of adhesive | None | None | None | Small | Small | Large |
| Protrusion height: 0 ram Hot pressing: 1.4 seconds | | | | | | |

**Table 9**

| | Heat Press Pressure (kg) | | | | | |
|---|---|---|---|---|---|---|
| | No Heat Press | 0.5 | 1.0 | 1.5 | 2.0 | 3 |
| Peel strength (g/width of electrode: 34 mm) | 118 | -- | 160 | 170 | 222 | -- |
| Squeeze-out amount of adhesive | None | -- | None | None | None | Large |
| Protrusion height: 0.4 mm Hot pressing: 1.4 seconds | | | | | | |

**Table 10**

| | Heat Press Pressure (kg) | | | | | |
|---|---|---|---|---|---|---|
| | No Heat Press | 0.5 | 1.0 | 1.5 | 2.0 | 3 |
| Peel strength (g/width of electrode: 34 mm) | 118 | -- | -- | 185 | 215 | -- |
| Squeeze-out amount of adhesive | None | -- | -- | None | None | Large |
| Protrusion height: 0.6 mm Hot pressing: 1.4 seconds | | | | | | |

When a heat block having no protrusion for forming a groove was used, the adhesion strength was improved up to 1 kg/cm². When the pressure was higher than 1 kg/cm², the adhesive was broken and squeezed out from the periphery of the electrode. When a heat block having a protrusion was used, the adhesion strength was improved at heat press pressures up to 2 kg/cm². However, when the pressure was increased to 3 kg/cm², a large amount of the adhesive was squeezed out from the periphery of the electrode.

A hot-pressed electrode and an identical electrode made without hot pressing (both made using the adhesive of Example 1), were applied to the lower arm of a human body and pressed by a 2 kg. Then a lead wire was attached to the tab portion of each electrode and a weight of 100 g was suspended from the lead wire. The subject operated an ergometer (bicycle for stress test) under a load of 100 W. The time until the electrodes peeled off was measured. The average peeling time for the hot-pressed electrodes was 12.5 minutes while the average peeling time of the electrodes prepared without hot pressing was 10.5 minutes.

## Claims

1. An electrode (40) adapted for attachment to an adherend comprising:
(a) an electrode support (41) having a first and a second opposed surface,
(b) a conductor (44) supported by the electrode support, and
(c) a conductive adhesive layer comprising a hydrophilic phase and a hydrophobic phase that is disposed upon a major portion of the first, opposed surface of the electrode support and the conductor, wherein a portion (26) of the conductive adhesive layer is hot-pressed to enhance the adhesion strength of the portion to an adherend, **characterized in that** another portion (48) of the conductive adhesive layer is not hot-pressed.

2. An electrode according to claim 1, wherein the conductor is either in the form of a layer of conductive material disposed upon at least a portion of the first, opposed surface of the electrode support, or, the conductor is in the form a conductive stud that passes through a portion of the first, opposed surface of the electrode support and completely through the electrode support to project below the second, opposed surface of the electrode support.

3. An electrode according to any preceding claim, wherein the electrode further comprises a release·liner disposed upon an exposed surface of the conductive adhesive layer.

4. The electrode according to any preceding claim, wherein the portion of the conductive adhesive layer that has been hot-pressed is a perimetrical portion.

5. The electrode according to any of the preceding claims, further comprising an indentation that separates the heat-pressed perimetral portion of the conductive adhesive layer from the portion of the conductive adhesive layer that has not been hot-pressed.

6. An electrode according to any preceding claim, wherein the conductor is in the form of a layer of conductive material and the area of the conductor is essentially coextensive with the area of the electrode support.

7. An electrode according to any preceding claim, wherein the conductor comprises a conductive ink.

8. An electrode according to any preceding claim, wherein the conductive adhesive layer comprises:
(a) a hydrophilic phase composing hydrophilic polymer material, an electrolyte, and a humectant, and
(b) a hydrophobic phase comprising hydrophobic polymer derived from the polymerization of hydrophobic monomer or oligomer in the presence of a surfactant and the hydrophilic phase.

9. An electrode according to claim 8, wherein the conductive adhesive layer consists essentially of:
(a) a hydrophilic phase comprising hydrophilic polymer material, an electrolyte, and a humectant, and
(b) a hydrophobic phase comprising hydrophobic polymer derived from the polymerization of hydrophobic monomer or oligomer in the presence of a surfactant and the hydrophilic phase.

10. An electrode according to any of claims 8 to 9, wherein the hydrophilic polymer material is selected from the group consisting of polymers containing one or more polyethylene glycol groups or polymers containing one or more pyrrolidone groups.

11. An electrode according to any of claims 8 to 10, wherein the electrolyte is selected from the group consisting of aqueous solutions of potassium chloride, sodium chloride or lithium chloride.

12. An electrode according to any of claims 8 to 11, wherein the humectant is selected from the group consisting of propylene glycol or sodium DL-pyrrolidonecarboxylate.

13. An electrode according to any of claims 8 to 12, wherein the hydrophobic polymer comprises interpolymerized units derived from one or more of the following monomers: acrylic acid, isooctyl acrylate, 2-ethylhexyl acrylate and n-butyl acrylate.

14. An electrode according to any of the preceding claims wherein the adherend is mammalian skin.

15. A method of improving the adhesion strength of a portion of a conductive adhesive layer comprising a hydrophilic phase and a hydrophobic phase by hot-pressing a portion of the conductive adhesive layer, **characterized in that** the method comprises the step of not hot-pressing another portion of the conductive adhesive layer.

16. The method according to claim 15, wherein the hot-pressed portion is a perimetrical portion.

17. The method according to any of claims 15 to 16, further comprising forming pinholes in the portion of the conductive adhesive layer to be hot-pressed prior to hot pressing.

## Patentansprüche

1. Elektrode (40), die zur Anbringung an einer Klebefläche geeignet ist, umfassend
(a) einen Elektrodenträger (41), der eine erste und eine zweite Oberfläche aufweist, die einander gegenüberliegen,
(b) einen Leiter (44), der durch den Elektrodenträger getragen wird, und
(c) eine leitfähige Klebstoffschicht, umfassend eine hydrophile Phase und eine hydrophobe Phase, die auf einem größeren Abschnitt der ersten, gegenüberliegenden Oberfläche des Elektrodenträgers und des Leiters angeordnet ist, wobei ein Abschnitt (26) der leitfähigen Klebstoffschicht heißgepreßt ist, um die Haftfestigkeit des Abschnitts an einer Klebefläche zu steigern, **dadurch gekennzeichnet, daß** ein weiterer Abschnitt (48) der leitfähigen Klebstoffschicht nicht heißgepreßt ist.

2. Elektrode nach Anspruch 1, wobei der Leiter entweder in der Form einer Schicht aus einem leitfähigen Material ausgeführt ist, das auf zumindest einem Abschnitt der ersten, gegenüberliegenden Oberfläche des Elektrodenträgers angeordnet ist, oder der Leiter in der Form eines leitfähigen Kontaktstifts ausgeführt ist, der durch einen Abschnitt der ersten, gegenüberliegenden Oberfläche des Elektrodenträgers und vollständig durch den Elektrodenträger verläuft, um unter der zweiten, gegenüberliegenden Oberfläche des Elektrodenträgers hervorzuragen.

3. Elektrode nach einem der vorhergehenden Ansprüche, wobei die Elektrode ferner eine Trenndeckschicht umfasst, die auf einer freiliegenden Oberfläche der leitfähigen Klebstoffschicht angeordnet ist.

4. Elektrode nach einem der vorhergehenden Ansprüche, wobei der Abschnitt der leitfähigen Klebstoffschicht, der heißgepreßt wurde, ein Umfangsabschnitt ist.

5. Elektrode nach einem der vorhergehenden Ansprüche, ferner umfassend eine Einkerbung, die den heißgepreßten Umfangsabschnitt der leitfähigen Klebstoffschicht von jenem Abschnitt der leitfähigen Klebstoffschicht trennt, der nicht heißgepreßt wurde.

6. Elektrode nach einem der vorhergehenden Ansprüche, wobei der Leiter in der Form einer Schicht aus einem leitfähigen Material ausgeführt ist, und die Fläche des Leiters im Wesentlichen koextensiv mit der Fläche des Elektrodenträgers ist.

7. Elektrode nach einem der vorhergehenden Ansprüche, wobei der Leiter eine leitfähige Tinte umfasst.

8. Elektrode nach einem der vorhergehenden Ansprüche, wobei die leitfähige Klebstoffschicht umfasst:
(a) eine hydrophile Phase, die ein hydrophiles Polymermaterial, einen Elektrolyt, und ein Feuchthaltemittel umfasst, und
(b) eine hydrophobe Phase, die ein hydrophobes Polymer umfasst, das aus der Polymerisation eines hydrophoben Monomers oder Oligomers in Anwesenheit eines oberflächenaktiven Mittels und der hydrophilen Phase erlangt wird.

9. Elektrode nach Anspruch 8, wobei die leitfähige Klebstoffschicht im Wesentlichen aus dem Folgenden besteht:
(a) einer hydrophilen Phase, die ein hydrophiles Polymermaterial, einen Elektrolyt, und ein Feuchthaltemittel umfasst, und
(b) einer hydrophoben Phase, die ein hydrophobes Polymer umfasst, das aus der Polymerisation eines hydrophoben Monomers oder Oligomers in Anwesenheit eines oberflächenaktiven Mittels und der hydrophilen Phase erlangt wird.

10. Elektrode nach einem der Ansprüche 8 bis 9, wobei das hydrophile Polymermaterial aus der Gruppe gewählt ist, die aus Polymeren, welche eine oder mehrere Polyethylenglykolgruppen enthalten, oder Polymeren, die eine oder mehrere Pyrrolidongruppen enthalten, besteht.

11. Elektrode nach einem der Ansprüche 8 bis 10, wobei der Elektrolyt aus der Gruppe gewählt ist, die aus wäßrigen Lösungen von Kaliumchlorid, Natriumchlorid oder Lithiumchlorid besteht.

12. Elektrode nach einem der Ansprüche 8 bis 11, wobei das Feuchthaltemittel aus der Gruppe gewählt ist, die aus Propylenglykol oder Natrium-DL-Pyrrolidoncarboxylat besteht.

13. Elektrode nach einem der Ansprüche 8 bis 12, wobei das hydrophobe Polymer mischpolymerisierte Einheiten umfasst, die von einem oder mehreren der folgenden Monomere erhalten werden: Acrylsäure, Isooctylacrylat, 2-Ethylhexylacrylat und n-Butylacrylat.

14. Elektrode nach einem der vorhergehenden Ansprüche,
wobei die Klebefläche Säugerhaut ist.

15. Verfahren zum Verbessern der Haftfestigkeit eines Abschnitts einer leitfähigen Klebstoffschicht, die eine hydrophile Phase und eine hydrophobe Phase umfasst, durch Heißpressen eines Abschnitts der leitfähigen Klebstoffschicht, **dadurch gekennzeichnet, daß** das Verfahren den Schritt des Nicht-Heißpressens eines weiteren Abschnitts der leitfähigen Klebstoffschicht umfasst.

16. Verfahren nach Anspruch 15, wobei der heißgepreßte Abschnitt ein Umfangsabschnitt ist.

17. Verfahren nach einem der Ansprüche 15 bis 16, ferner umfassend ein dem Heißpressen vorausgehendes Bilden von Nadellöchern im heißzupressenden Abschnitt der leitfähigen Klebstoffschicht.

## Revendications

1. Electrode (40) adaptée pour être fixée à une base d'adhérence et qui comprend:
(a) un support d'électrode (41) qui présente une première et une deuxième surface opposées,
(b) un conducteur (44) soutenu par le support d'électrode, et
(c) une couche adhésive conductrice qui comprend une phase hydrophile et une phase hydrophobe, disposée sur une partie majeure de la première surface opposée du support d'électrode et du conducteur, une partie (26) de la couche adhésive conductrice étant pressée à chaud pour renforcer la force d'adhérence de la partie à une base d'adhérence, **caractérisée en ce qu'**une autre partie (48) de la couche adhésive conductrice n'est pas pressée à chaud.

2. Electrode selon la revendication 1, dans laquelle le conducteur présente soit la forme d'une couche de matériau conducteur disposée sur au moins une partie de la première surface opposée du support d'électrode soit la forme d'un tourillon conducteur qui traverse une partie de la première surface opposée du support d'électrode et qui traverse complètement le support d'électrode pour déborder en dessous de la deuxième surface opposée du support d'électrode.

3. Electrode selon l'une quelconque des revendications précédentes, dans laquelle l'électrode comprend en outre une garniture de libération disposée sur une surface exposée de la couche adhésive conductrice.

4. Electrode selon l'une quelconque des revendications précédentes, dans laquelle la partie de la couche adhésive conductrice qui a été pressée à chaud est une partie périphérique.

5. Electrode selon l'une quelconque des revendications précédentes, qui comprend en outre une indentation qui sépare la partie périphérique pressée à chaud de la couche adhésive conductrice de la partie de la couche adhésive conductrice qui n'a pas été pressée à chaud.

6. Electrode selon l'une quelconque des revendications précédentes, dans laquelle le conducteur présente la forme d'une couche de matériau conducteur, la surface du conducteur s'étendant essentiellement sur toute la surface du support d'électrode.

7. Electrode selon l'une quelconque des revendications précédentes, dans laquelle le conducteur comprend une ancre conductrice.

8. Electrode selon l'une quelconque des revendications précédentes, dans laquelle la couche adhésive conductrice comprend:
(a) une phase hydrophile qui comprend un matériau polymère hydrophile, un électrolyte et un agent d'humidification, et
(b) une phase hydrophobe qui comprend un polymère hydrophobe obtenu par polymérisation d'un monomère ou oligomère hydrophobe en présence d'un agent tensioactif et de la phase hydrophile.

9. Electrode selon la revendication 8, dans laquelle la couche adhésive conductrice est essentiellement constituée:
(a) d'une phase hydrophile qui comprend un matériau polymère hydrophile, un électrolyte et un agent d'humidification, et
(b) d'une phase hydrophobe qui comprend un polymère hydrophobe obtenu par polymérisation d'un monomère ou oligomère hydrophobe en présence d'un agent tensioactif et de la phase hydrophile.

10. Electrode selon l'une quelconque des revendications 8 à 9, dans laquelle le matériau polymère hydrophile est sélectionné dans le groupe qui est constitué des polymères qui contiennent un ou plusieurs groupes poly(éthylène glycol) ou de polymères qui contiennent un ou plusieurs groupes pyrrolidone.

11. Electrode selon l'une quelconque des revendications 8 à 10, dans laquelle l'électrolyte est sélectionné dans le groupe constitué de solutions aqueuses de chlorure de potassium, de chlorure de sodium et de chlorure de lithium.

12. Electrode selon l'une quelconque des revendications 8 à 11, dans laquelle l'agent d'humidification est sélectionné dans le groupe constitué du propylène glycol et du DL-pyrrolidonecarboxylate de sodium.

13. Electrode selon l'une quelconque des revendications 8 à 12, dans laquelle le polymère hydrophobe comprend des unités interpolymérisées obtenues à partir d'un ou plusieurs monomères parmi les monomères suivants: acide acrylique, acrylate d'isooctyle, acrylate de 2-éthylhexyle et acrylate de n-butyle.

14. Electrode selon l'une quelconque des revendications précédentes, dans laquelle la base d'adhérence est la peau d'un mammifère.

15. Procédé d'amélioration de la force d'adhérence d'une partie d'une couche adhésive conductrice comprenant une phase hydrophile et une phase hydrophobe par pressage à chaud d'une partie de la couche adhésive conductrice, **caractérisé en ce que** le procédé comprend l'étape qui consiste à ne pas presser à chaud une autre partie de la couche adhésive conductrice.

16. Procédé selon la revendication 15, dans lequel la partie pressée à chaud est une partie périphérique.

17. Procédé selon l'une quelconque des revendications 15 à 16, qui comprend en outre la formation de petits trous dans la partie de la couche adhésive conductrice qui doit être pressée à chaud, et ce avant le pressage à chaud.
